# EUROPEAN PATENT SPECIFICATION

(11) **EP 1 424 004 B1**
(45) Date of publication and mention of the grant of the patent: **16.01.2008**
(21) Application number: 03026721.5
(22) Date of filing: 21.11.2003
(51) Int. Cl.: A01G 13/02

(54) **Weed-preventing paper for use in plant cultivation**
Papier zum Verhindern von Unkraut für die Verwendung bei der Pflanzenzucht
Papier pour la prévention des herbes mauvaises dans la culture de plantes

(30) Priority: 26.11.2002 CN 02152644
(43) Date of publication of application: 02.06.2004
(73) Proprietor: YUEN FOONG YU BIO-TECH CO., LTD., Taipei City 100 (TW)
(72) Inventor: Huang, Rui-Zhi, Taoyuan Taiwan, R.O.C. (TW); Chou, Chun-Chi, Sin-Shih Tounship Tainan County, R.O.C. (TW)
(74) Representative: Reinhard - Skuhra - Weise & Partner

(56) References cited:
- FR-A- 2 650 149
- GB-A- 2 343 606
- US-A- 2 249 867
- US-A- 3 940 884
- US-A- 4 063 452

## Description

The present invention relates to a weed-preventing paper for cultivating a plant, and more particularly to a multi-functional weed-preventing paper with a cross opening for a plant to pass therethrough, a control-release fertilizer, a beautifying figure and a water indicator.

The horticultural crops include flowers, fruiters, garden vegetable and special crops and so on. There are many problems causing from weeds during the cultivating period. Because the growing period for the perennial fruiter is very long, the competitions between fruiters and weeds mostly occur at any time. Most parts of the cultivating area locate on the mountainside and the cultivating area is usually very wide. Thus, the weed control has become more and more difficult. The farmers usually remove the weeds under canopy area for conveniently applying the fertilizer to the soil and proceeding the further productive management. One of the common ways for proceeding the weed control is to use the herbicides. As we know, the chemical reagents are harmful to both the environment and the people. And, some crops are sensitive to certain reagents so that we can't use the chemical reagents to proceed the weed control sometimes. As a result, a number of manpower are needed to remove the weeds by hand. However, the step of hand weeding is the most time-consuming process, which always delays the agricultural production for a period of time and increases the production costs.

Recently, the quality life is gradually improved and thus home gardening has become more and more popular for beautifying the environment and enhancing the life interest. However, there also exist weed control problems and watering management for the plant growth. These always bother the owners of the pot plants. It will easily cause the plant diseases if the soil is too wet. On the contrary, the plant would wither when the soil is too dry. Hence, it is a big concern for the plant owner about how to water the pot plant at suitable time.

There are several known schemes used for the weed removal or the water management of the soil. For example, the US Patent No. 3,940,884 discloses a universal soil cover for use with plants in different sized containers including a sheet capable of retaining water, a plastic film adhered to the upper surface of the water-retaining sheet. The sheet and film have openings for receiving a plant stem, and also have a plurality of rows of spaced perforations adapted to permit ready tearing away of outer portion of the cover, in order to reduce the cover to a size conforming to a container holding the plant.

US Patent No. 4,063,452 discloses a water indicator enclosed in an envelope. The envelope includes a transparent cover and base, each of which is formed by the polymeric material, and the indicator is placed adjacent a plant.

However, the opening of the paper body disclosed in US Patent No. 3,940,884 is in the center of the disc, which is not convenient to be used in general planting case since the stem of the plant is not always in the center of the flower pot. On the other hand, the indicator disclosed in the US Patent No. 4,063,452 may be interfered by the mentioned paper body.

From the above description, it is known that how to develop a method for satisfying the requirements of weed removal and providing a good water management has become a major problem waited to be solved. In order to satisfy the needs described above, a weed-preventing paper for cultivating a plant is provided. The particular design in the present invention uses a paper not only containing a weed-preventing agent for preventing the weed growth, but also including a water indicator for reminding the plant owner to water the plant. It is very convenient and easy for use, and therefore the man-power and production costs are greatly reduced. Thus, the invention has the utility for the agricultural industry and home gardening.

Therefore, the present invention provides a weed-preventing paper for cultivating a plant which overcomes the disadvantages described above.

Another aspect, character and executive adduction of the present invention will become more completely comprehensible by the following revelation and accompanying claim.

It is an object of the present invention to provide a multi-purpose agricultural paper having the features of biodegrading and strong light-blocking ability so that it can be used as functional basic materials to control weeds, indicator to water plants, and supply fertilizer, and most of all, it saves manpower.

It is therefore an object of the present invention to provide a weed-preventing paper for use in plant cultivation for effectively preventing the weed growth.

It is another object of the present invention to provide a weed-preventing paper for use in plant cultivation. The weed-preventing paper has specific cross openings for a plant conveniently to pass therethrough.

It is another object of the present invention to provide a weed-preventing paper for use in plant cultivation. The weed-preventing paper with the design of watering indication could provide the function of reminding the owner of the pot plant to water the pot plant timely.

In accordance with an aspect of the present invention, the weed-preventing paper for cultivating a plant includes a paper body covering a surface of a soil where the plant is to be grown and having at least a cross opening for the plant to pass therethrough, a weed-preventing agent contained in the paper body for blocking light so as to prevent the growth of a weed surrounding the plant, and a water indicator printed on the paper body and showing a hydrous and an anhydrous states thereof via different colors for being a reminder of watering.

Preferably, the cross opening has a lateral crosscut extending to an edge of the paper body for facilitating the paper body to be mounted around a stem of the plant.

Preferably, the water indicator contains a cobaltous chloride (CoCl₂).

Preferably, the water indicator is printed on the paper in a shape of a word or a figure.

Preferably, the weed-preventing paper further includes a control-release fertilizer to be dissolvable in water to provide nutrients for the growth of the plant and reduce the manpower cost of fertilizing.

Preferably, the control-release fertilizer is integrated into the paper body by adding in a pulp thereof, or coated thereon or adhered thereto.

Preferably, the weed-preventing paper further includes a beautifying figure printed on the paper body.

Preferably, the beautifying figure is an image selected from a group consisting of a flower, a grass, a person and a scenery.

Preferably, a surface of the paper body has a silver color for preventing bugs.

Preferably, the weed-preventing paper is applied to one of a potted plant and a fruit tree.

Preferably, the paper body is circular.

Preferably, the paper body has a diameter equal to that of one of a pot where the potted plant is grown therein and an under-canopy area of the fruit tree.

Preferably, the weed-preventing agent is one of inorganic and organic fillers being one selected from a group consisting of a black carbon, a silicon dioxide and a titanium dioxide.

Preferably, the paper body is made of a biodegradable fiber.

Preferably, the fiber is one of a plant fiber and a polymer fiber.

The above objects and advantages of the present invention will become more readily apparent to those ordinarily skilled in the art after reviewing the following detailed descriptions and accompanying drawings, in which:

Figs. 1(a) and 1(b) are the schematic diagrams showing the weed-preventing paper with a cross opening according to a preferred embodiment of the present invention.

Figs. 2(a) and 2(b) are the schematic diagrams showing the weed-preventing paper with a water indicator according to a preferred embodiment of the present invention, wherein Fig. 2(a) is the weed-preventing paper before watering and Fig. 2(b) is the weed-preventing paper after watering.

Fig. 3 shows the practical application of the watering indication design according to a preferred embodiment of the present invention.

Fig. 4 is the schematic diagram showing the weed-preventing paper according to a preferred embodiment of the present invention.

Figs. 5(a) and 5(b) are the schematic diagrams showing the weed-preventing paper according to a preferred embodiment of the present invention.

The foregoing and other features and advantages of the present invention will be more clearly understood through the following descriptions with reference to the drawings, wherein:

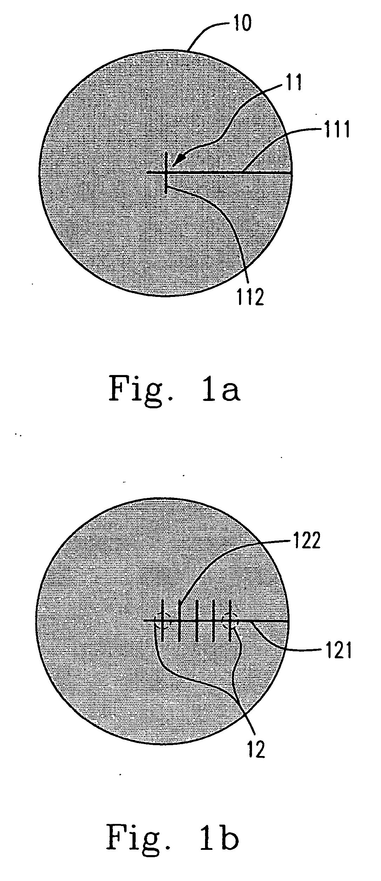

Please refer to Fig. 1a which is the schematic diagram showing the weed-preventing paper with a cross opening according to a preferred embodiment of the present invention. The weed-preventing paper includes a paper body 10 which is composed of a biodegradable material, such as plant fibers or polymer fibers. Thus, the weed-preventing paper has the advantage of being decomposed in natural environment easily and does not result in environmental pollution. Moreover, the paper body 10 includes a weed-preventing agent for preventing the weed growth. The weed-preventing agent can be an inorganic or organic filler including black carbon, silicon dioxide or titanium dioxide. The light is blocked out up to 100 % by the paper body 10 containing a weed-preventing agent. Thus, the weeds die resulting from the weed seeds being incapable of germinating or the weed seedlings being incapable of carrying out photosynthesis under dark condition so that the weed growth surrounding the plant is inhibited efficiently.

The weed-preventing paper according to the present invention could be applied to a pot plant. The weed-preventing paper is cut into round shape having a diameter equal to that of the pot so as to fit the pot size, and a cross opening 11 is formed at the central portion of the paper. The cross opening 11 consists of a long cutting opening 111 and a short cutting opening 112 vertical to the long one. The long cutting opening 111 extends from the central portion to an edge of the paper body 10. A plant can pass through the cross opening 11 which facilitates the paper body 10 to be mounted around the stem of the plant. Therefore, the weed control is achieved by covering the soil surface of the pot with the weed-preventing paper of the present invention.

Please refer to Fig. 1b which is the schematic diagram showing the weed-preventing paper with a cross opening according to a preferred embodiment of the present invention. Because the deviation of the planting position of the pot plant might occur due to man-made planting or natural growing, the position of the pot plant is not always positioned in the central portion of the pot. That is, the weed-preventing paper is applied to the pot plant planted in the central portion of the pot for passing therethrough only if a cross opening is formed at the central portion thereof. In order to overcome this defect, the design of the weed-preventing paper according to a preferred embodiment of the present invention is that an opening is cut from the center to the edge of the paper to be a basic cutting opening 121 and a plurality of cutting openings 122 with suitable lengths are cut vertically thereto so as to form multiple cross openings 12 on the weed-preventing paper. Hence, the weed-preventing paper could be used for properly covering the soil surface no matter where the plant is planted.

Besides, the present invention also has the design of watering indication for assisting the owner of the pot plant to do the water management in order to timely water the pot plant. The design of watering indication is to use a water indicator printed on the paper body, which shows a hydrous and an anhydrous states via different colors for being a reminder of watering. One of the water indicators suitable for use in the present invention is cobaltous chloride (CoCl₂). Cobaltous chloride is a crystalline compound whose anhydrous form consists of blue crystals, and whose hydrous form consists of pink crystals. That is to say the color of cobaltous chloride is blue while it is dry, and is pink while it absorbs water to contain crystal water.

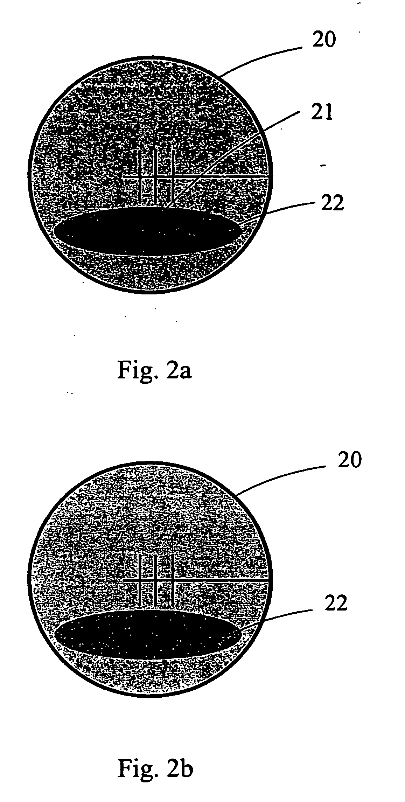

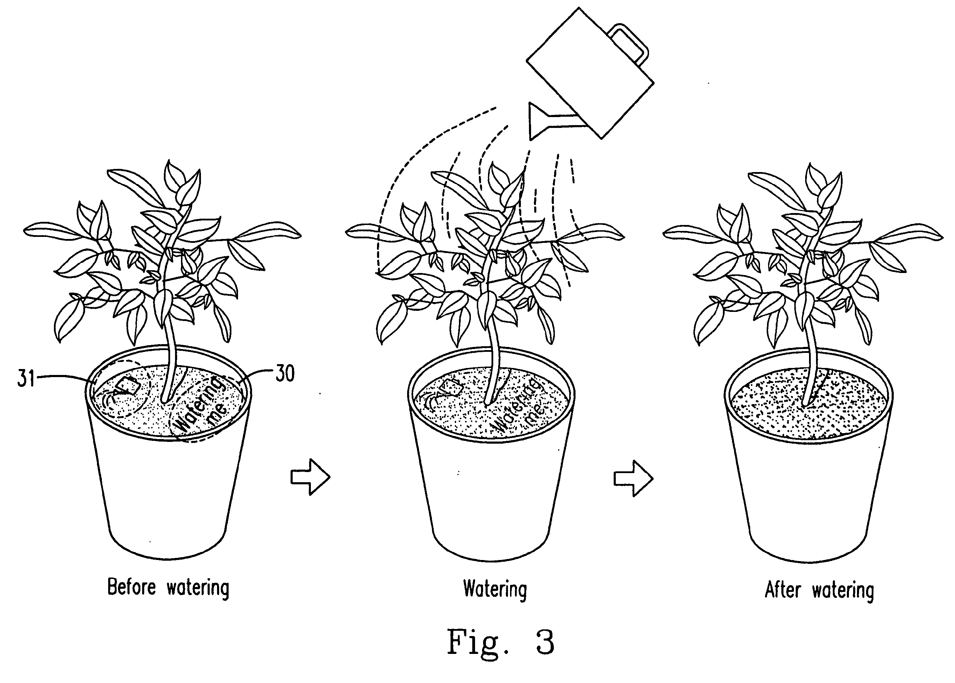

Please refer to Figs. 2a and 2b which show the weed-preventing paper with a water indicator according to a preferred embodiment of the present invention. According to a preferred embodiment of the present invention, cobaltous chloride is printed on the paper body 20 of the weed-preventing paper in the form of reminding words 21 (as shown in Fig. 2a) or figures 31 (as shown in Fig. 3). The reminding words 21 could be the words, for example, "Watering Me" and "Give me some water" and so on. The weed-preventing paper is attached close to the soil surface of the pot. While the weed-preventing paper is in a dry manner due to the soil lacks of water, CoCl₂ printed thereon does not contain crystal water and thus becomes blue (as shown in Fig. 2a). On the contrary, the weed-preventing paper will absorb water while the soil is wet after watering, and consequently the CoCl₂ will also absorb water to contain crystal water and thus become pink. Therefore, the background 22 of the reminding words 21 (as shown in Fig. 2a) could be printed with a pink color. In such a way, the reminding words 21 show the blue color when the soil is dry, which reveals the big difference compared with the background 22 with pink color so as to produce the effect for reminding the plant owner to water the plant. However, the reminding words 21 reveal pink color while the soil is wet, that is the same as that of the background 22 to let those words or figures hidden (as shown in Fig. 2b).

Please refer to Fig. 3 which shows the implementation of the watering reminding design of the weed-preventing paper according the present invention. There is no need to limit the color of the background of the weed-preventing paper to be pink because the color of CoCl₂ is almost colorless at low concentration (< 0.6 M). Referring to Fig. 3, it could be seen that the printed reminding words 30 or figures 31 could be clearly presented for reminding the plant owner to water the plant when the soil of the pot plant is dry. After the pot plant is watered, the color of the reminding words 30 or figures 31 is nearly colorless, which shows the effect of hiding the reminding words 30 or figures 31. From the description described above, the weed-preventing paper of the present invention has the function of reminding the pot plant owner to water the pot plant via the water indicator while the soil of the pot plant is too dry. Besides, the pot plant owner could be aware of the water content in the soil of the pot plant and decide whether to water the pot plant or not to avoid improper water management for the pot plant, which leads to lead to the death of the plant.

According to a preferred embodiment of the present invention, the weed-preventing paper further includes a control-release fertilizer which is an organic or chemical fertilizer (such as the "MAGAMP" in the market). The control-release fertilizer can dissolve in water slowly. The nutrients are released in the water and then provided to the pot plant for growth requirement so as to reduce the manpower of manually fertilization. The control-release fertilizer is integrated into the paper body by being mixed with the pulp, or coated on or adhered to the paper body.

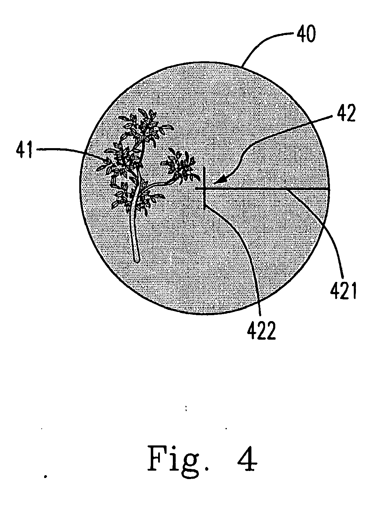

Please refer to Fig. 4 which shows the weed-preventing paper according to a preferred embodiment of the present invention. According to the preferred embodiment of the present invention, the weed-preventing paper further includes a beautifying figure 41 printed on the paper body 40 for beautifying the soil surface of the pot plant, such as an image of a flower, a grass, a person or a scenery. A silver color could also be printed on a surface of the paper body 40 for producing the effect of preventing bugs as a silver plastic sheet does.

Additionally, the weed-preventing paper could also be applied to the weed control under canopy area of the fruit three. Please refer to Fig. 4 again. The paper body 40 of the weed-preventing paper for use in the fruit tree is made of waste paper sludge and the control-release fertilizer for fruit tree is added thereto. The weight of the paper body 40 of the weed-preventing paper is increased to avoid the paper body 40 from being blown in the wind and thus the weed-preventing paper is well attached to the soil surface of the under-canopy area of the fruit tree. The paper body 40 is cut into a circular shape with a diameter equal to that of the under-canopy area of the fruit tree. A cross opening 42 is formed at the central portion of the paper body 40, so that the stem of the fruit tree can pass therethrough easily. The cross opening 42 has a long cutting opening 421 cut from the central to the edge of the paper body 40 and at least one short cutting opening 422 vertical to the long one. The cross opening 42 is used for facilitating the paper body 40 to be mounted around the stem of the fruit tree so that the weed-preventing paper covers the soil surface of the under-canopy area of the fruit tree for preventing the weeds and managing the fertilization. A beautifying figure 41 for beautifying the soil surface of the under-canopy area of the fruit tree is printed on the paper body 40 too, for example, an image of a flower, a grass, a person or a scenery and so on. Since the fruits are often ate or plucked by the animal, a figure with deterrent use (such as an image of an eagle etc.) can be printed on the paper body 40 for frightening animals away. Also, the beautifying figure 41 can make the soil surface look pleasant after fertilization treatment.

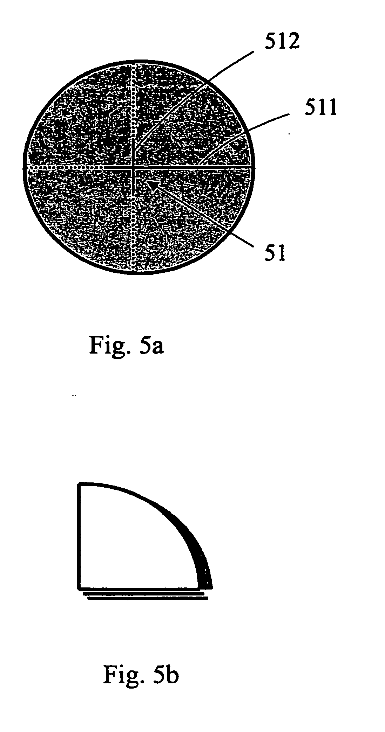

Please refer to Fig. 5a and 5b which are the schematic diagrams showing the weed-preventing paper for the under-canopy area of the fruit tree according to a preferred embodiment of the present invention. As shown in Fig. 5a, the weed-preventing paper has a circular shape and a cross opening 51. The cross opening 51 has a long cutting opening 511, a short cutting opening 512 vertical to the long one, and pre-folded folds (the dotted lines in Fig. 5b). The pre-folded folds of the weed-preventing paper are formed by folding up the paper based on the long cutting opening 511 and then folding up the paper again. The weed-preventing paper with folded form is shown in Fig. 5(b). The folded weed-preventing paper can be conveniently stored, transited, carried and operated.

The weed preventing test using the weed-preventing paper of the present invention is as follows. A 7-inch-diameter pot filled with proper amount of growing media (soil : peat moss = 3 : 1, v/v) is provided and a coreopsis (*Corepsis tinctoria* Nutt.) seedling is transplanted thereto. Then, the seedling is passed through the opening of the weed-preventing paper and thus the soil surface is covered by the paper. After the foregoing steps, the pot plant is moved to the outdoors for further cultivation. A pot plant without the covering of weed-preventing paper is the control. Each treatment is set in six repetitions. The weed growth is observed and recorded during the cultivation. The results are shown in Table 1

**Table 1**

| | Average weed No. (plant) | | | | | | |
|---|---|---|---|---|---|---|---|
| Treatment | Cultivating week(s) | | | | | | |
| | 1 | 2 | 3 | 4 | 8 | 12 | 24 |
| weed-preventing paper | 0.0 | 0.0 | 0.0 | 0.0 | 0.0 | 0.8 | 0.8 |
| Control | 23.3 | 48.2 | 53.8 | 54.8 | 51.2 | 43.0 | 40.5 |

As shown in Table 1, the average weed number for the treatment of pot-cultured coreopsis covered with weed-preventing paper is 2.5 after 24 weeks cultivation and that of the control is 40.5. There are significantly different weed numbers between these two treatments after same cultivation periods. Therefore, it is an effective method to prevent the weed growth by using the weed-preventing paper of the present invention.

According to the above, it is clear that the weed-preventing paper for use in plant cultivation of the present invention has the feature of being capable of effectively preventing the weed growth. The weed-preventing paper has multiple cross openings for passing a plant therethrough, a silver color for preventing bugs, the control-release fertilizer for saving the manpower cost, the beautifying image for beautifying the soil surface and a water indicator for reminding the plant owner to do the water management and so on. A certain combination of the features described above could be made depending on the user's needs. In addition, all the related materials in the present invention are biodegradable and capable of being decomposed, which is not limited by the soil environment and enables the achievement of the permanent operation in agricultural production. Therefore, the present invention has the features of industrial application and high commercial value.

## Claims

1. A weed-preventing paper for cultivating a plant, comprising:
a paper body (10, 20, 40) covering a surface of a soil where the plant is to be grown, and having at least a cross opening (11, 12, 42, 51) for the plant to pass therethrough;
a weed-preventing agent contained in the paper body (10, 20, 40) for blocking light so as to prevent the growth of a weed surrounding the plant;
**characterized in that** the weed-preventing paper comprises a water indicator printed on the paper body (10, 20, 40) and showing a hydrous and an anhydrous states thereof via different colors for being a reminder of watering.

2. The weed-preventing paper as claimed in claim 1 **characterized in that** the cross opening (11, 12, 42, 51) has a lateral crosscut extending to an edge of the paper body (10, 20, 40) for facilitating the paper body (10, 20, 40) to be mounted around a stem of the plant.

3. The weed-preventing paper as claimed in claim 1 or 2 **characterized in that** the water indicator contains a cobaltous chloride (CoCl₂).

4. The weed-preventing paper as claimed in one of the preceding claims **characterized in that** the water indicator is printed on the paper in a shape of a word (21, 30) or a figure (31).

5. The weed-preventing paper as claimed in one of the preceding claims **characterized by** comprising a control-release fertilizer to be dissolvable in water to provide nutrients for the growth of the plant and reduce the manpower cost of fertilizing.

6. The weed-preventing paper as claimed in one of the preceding claims **characterized in that** the control-release fertilizer is integrated into the paper body (10, 20, 40) by adding in a pulp thereof, or coated thereon or adhered thereto.

7. The weed-preventing paper as claimed in one of the preceding claims **characterized** comprising a beautifying figure (41) printed on the paper body (10, 40).

8. The weed-preventing paper as claimed in one of the preceding claims **characterized in that** the beautifying figure (41) is an image selected from a group consisting of a flower, a grass, a person and a scenery.

9. The weed-preventing paper as claimed in one of the preceding claims **characterized in that** a surface of the paper body (10, 20, 40) has a silver color for preventing bugs.

10. The weed-preventing paper as claimed in one of the preceding claims **characterized in that** the weed-preventing paper is applied to one of a potted plant and a fruit tree.

11. The weed-preventing paper as claimed in one of the preceding claims **characterized in that** the paper body (10, 20, 40) is circular.

12. The weed-preventing paper as claimed in one of the preceding claims **characterized in that** the paper body (10, 20, 40) has a diameter equal to that of one of a pot where the potted plant is grown therein and an under-canopy area of the fruit tree.

13. The weed-preventing paper as claimed in one of the preceding claims **characterized in that** the weed-preventing agent is one of inorganic and organic fillers being one selected from a group consisting of a black carbon, a silicon dioxide and a titanium dioxide.

14. The weed-preventing paper as claimed in one of the preceding claims **characterized in that** the paper body (10, 20, 40) is made of a biodegradable fiber.

15. The weed-preventing paper as claimed in one of the preceding claims **characterized in that** the fiber is one of a plant fiber and a polymer fiber.

## Patentansprüche

1. Papier zum Verhindern von Unkraut zum Kultivieren einer Pflanze, umfassend:
einen Körper aus Papier (10,20,40), der eine Oberfläche eines Bodens, auf dem die Pflanze angezüchtet werden soll, bedeckt und wenigstens eine kreuzförmige Öffnung (11, 12, 42, 51) aufweist, durch die die Pflanze hindurchgehen kann;
ein Mittel zum Verhindern von Unkraut, das in dem Körper aus Papier (10, 20, 40) zum Blockieren von Licht enthalten ist, um so das Wachstum von Unkraut, das die Pflanze umgibt, zu verhindern;
**dadurch gekennzeichnet, dass** das Papier zum Verhindern von Unkraut einen auf den Körper aus Papier (10, 20, 40) aufgedruckten Wasserindikator umfasst, der einen wasserhaltigen und einen wasserfreien Zustand desselben über verschiedene Farben anzeigt und so eine Erinnerung für eine Bewässerung darstellt.

2. Papier zum Verhindern von Unkraut nach Anspruch 1, **dadurch gekennzeichnet, dass** die kreuzförmige Öffnung (11, 12, 42, 51) einen seitlichen Einschnitt aufweist, der bis zu einem Rand des Körpers aus Papier (10, 20, 40) reicht, um das Befestigen des Körpers aus Papier (10, 20, 40) um den Stamm der Pflanze herum zu erleichtern.

3. Papier zum Verhindern von Unkraut nach Anspruch 1 oder 2, **dadurch gekennzeichnet, dass** der Wasserindikator Cobaltchlorid (CoCl₂) enthält.

4. Papier zum Verhindern von Unkraut nach einem der vorhergehenden Ansprüche, **dadurch gekennzeichnet, dass** der Wasserindikator in Form eines Worts (21, 30) oder eines Zeichens (31) auf das Papier aufgedruckt wird.

5. Papier zum Verhindern von Unkraut nach einem der vorhergehenden Ansprüche, **dadurch gekennzeichnet, dass** es ein kontrolliert freisetzbares Düngemittel, das in Wasser lösbar sein soll und so Nährstoffe für das Wachstum der Pflanzen bereitstellt und die Personalkosten für das Düngen verringert, umfasst.

6. Papier zum Verhindern von Unkraut nach einem der vorhergehenden Ansprüche, **dadurch gekennzeichnet, dass** das kontrolliert freisetzbare Düngemittel in den Körper aus Papier integriert ist, indem es zu der Pulpe desselben gegeben wird, auf dieses aufgetragen wird oder mit diesem verklebt wird.

7. Papier zum Verhindern von Unkraut nach einem der vorhergehenden Ansprüche, **dadurch kennzeichnet, dass** es ein auf den Körper aus Papier aufgedrucktes, verschönerndes Zeichen (41) umfasst.

8. Papier zum Verhindern von Unkraut nach einem der vorhergehenden Ansprüche, **dadurch gekennzeichnet, dass** das verschönernde Zeichen (41) ein Bild ist, das ausgewählt ist aus einer Gruppe bestehend aus Blumen, Gras, einer Person und einer Landschaft.

9. Papier zum Verhindern von Unkraut nach einem der vorhergehenden Ansprüche, **dadurch gekennzeichnet, dass** eine Oberfläche des Körpers aus Papier eine silberne Farbe zum Abhalten von Käfern aufweist.

10. Papier zum Verhindern von Unkraut nach einem der vorhergehenden Ansprüche, **dadurch gekennzeichnet, dass** das Papier zum Verhindern von Unkraut bei einer eingetopften Pflanze oder einem Obstbaum angewendet wird.

11. Papier zum Verhindern von Unkraut nach einem der vorhergehenden Ansprüche, **dadurch gekennzeichnet, dass** der Körper aus Papier (10, 20, 40) kreisförmig ist.

12. Papier zum Verhindern von Unkraut nach einem der vorhergehenden Ansprüche, **dadurch gekennzeichnet, dass** der Körper aus Papier (10, 20, 40) einen Durchmesser aufweist, der genauso groß ist wie derjenige eines Topfs, in dem die eingetopfte Pflanze angezüchtet wird, und einer Fläche unterhalb der Bodenbedeckung des Obstbaums.

13. Papier zum Verhindern von Unkraut nach einem der vorhergehenden Ansprüche, **dadurch gekennzeichnet, dass** das Mittel zum Verhindern von Unkraut eines von einem anorganischen und organischen Füllstoff ist, der ausgewählt ist aus der Gruppe bestehend aus Carbon Black, einem Siliziumdioxid und einem Titandioxid.

14. Papier zum Verhindern von Unkraut nach einem der vorhergehenden Ansprüche, **dadurch gekennzeichnet, dass** der Körper aus Papier (10, 20, 40) aus einer biologisch abbaubaren Faser besteht.

15. Papier zum Verhindern von Unkraut nach einem der vorhergehenden Ansprüche, **dadurch gekennzeichnet, dass** die Faser eine Pflanzenfaser oder eine Polymerfaser ist.

## Revendications

1. Papier de lutte contre les mauvaises herbes pour cultiver une plante, comprenant :
un corps de papier (10, 20, 40) recouvrant une surface d'un sol où la plante doit être cultivée, et ayant au moins une ouverture transversale (11, 12, 42, 51) pour que la plante passe à travers ;
un agent de lutte contre les mauvaises herbes contenu dans le corps de papier (10, 20, 40) pour bloquer la lumière de manière à empêcher la croissance d'une mauvaise herbe entourant la plante ;
**caractérisé en ce que** le papier de lutte contre les mauvaises herbes comprend un indicateur d'eau imprimé sur le corps de papier (10, 20, 40) et représentant un état hydrique et un état anhydre de celui-ci par différentes couleurs destiné à être un rappel d'arrosage.

2. Papier de lutte contre les mauvaises herbes selon la revendication 1, **caractérisé en ce que** l'ouverture transversale (11, 12, 42, 51) possède une coupe transversale latérale s'étendant à un bord du corps de papier (10, 20, 40) pour faciliter le montage du corps de papier (10, 20, 40) autour d'une tige de la plante.

3. Papier de lutte contre les mauvaises herbes selon la revendication 1 ou 2, **caractérisé en ce que** l'indicateur d'eau contient un chlorure de cobalt (CoCl₂).

4. Papier de lutte contre les mauvaises herbes selon l'une des revendications précédentes, **caractérisé en ce que** l'indicateur d'eau est imprimé sur le papier sous une forme d'un mot (21, 30) ou d'un dessin (31).

5. Papier de lutte contre les mauvaises herbes selon l'une des revendications précédentes, **caractérisé en ce qu'**il comprend un engrais à libération contrôlée soluble dans l'eau pour fournir des nutriments pour la croissance de la plante et réduire le coût de main d'oeuvre pour l'apport d'engrais.

6. Papier de lutte contre les mauvaises herbes selon l'une des revendications précédentes, **caractérisé en ce que** l'engrais à libération contrôlée est intégré dans le corps de papier (10, 20, 40) par addition dans une pulpe de celui-ci, ou revêtu dessus ou collé à celui-ci.

7. Papier de lutte contre les mauvaises herbes selon l'une des revendications précédentes, **caractérisé en ce qu'**il comprend une figure de mise en valeur (41) imprimée sur le corps de papier (10, 40).

8. Papier de lutte contre les mauvaises herbes selon l'une des revendications précédentes, **caractérisé en ce que** la figure de mise en valeur (41) est une image choisie à partir d'un groupe consistant en une fleur, une plante, une personne et un décor.

9. Papier de lutte contre les mauvaises herbes selon l'une des revendications précédentes, **caractérisé en ce qu'**une surface du corps de papier (10, 20, 40) possède une couleur argentée pour lutter contre les insectes.

10. Papier de lutte contre les mauvaises herbes selon l'une des revendications précédentes, **caractérisé en ce que** le papier de lutte contre les mauvaises herbes est appliqué à l'un parmi une plante en pot et un arbre fruitier.

11. Papier de lutte contre les mauvaises herbes selon l'une des revendications précédentes, **caractérisé en ce que** le corps de papier (10, 20, 40) est circulaire.

12. Papier de lutte contre les mauvaises herbes selon l'une des revendications précédentes, **caractérisé en ce que** le corps de papier (10, 20, 40) a un diamètre égal à celui d'un pot où la plante en pot est cultivée et une région sous la canopée de l'arbre fruitier.

13. Papier de lutte contre les mauvaises herbes selon l'une des revendications précédentes, **caractérisé en ce que** l'agent de lutte contre les mauvaises herbes est une charge parmi une charge inorganique et organique choisie parmi un groupe consistant en un noir de charbon, un dioxyde de silicium et un dioxyde de titane.

14. Papier de lutte contre les mauvaises herbes selon l'une des revendications précédentes, **caractérisé en ce que** le corps de papier (10, 20, 40) est fait d'une fibre biodégradable.

15. Papier de lutte contre les mauvaises herbes selon l'une des revendications précédentes, **caractérisé en ce que** la fibre est une fibre parmi une fibre végétale et une fibre polymère.
